# EUROPEAN PATENT APPLICATION

(11) **EP 3 974 519 A1**
(43) Date of publication of application: **30.03.2022**
(21) Application number: 20809188.4
(22) Date of filing: 28.04.2020
(51) Int. Cl.: C12N 5/077, C12N 1/00

(54) **EXPANSION CULTURE METHOD FOR CARTILAGE OR BONE PRECURSOR CELLS**

(30) Priority: 20.05.2019 JP 2019094878
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); TOKUYAMA, Mayumi, Kawasaki-shi, Kanagawa 210-8681 (JP)
(74) Representative: Hoffmann Eitle
(86) International application number: PCT/JP2020/018130
(87) International publication number: WO 2020/235319

(57) **Abstract**

A method for expansion culture of a cartilage or bone progenitor cell, including a step of culturing a cartilage or bone progenitor cell in a medium containing a TGF-β signal inhibitor and FGF, and a method for producing a chondrocyte or osteocyte, including a step of inducing differentiation of a cartilage or bone progenitor cell obtained by the method into a chondrocyte or osteocytes are provided by the present invention.

## Description

### [Technical Field]

The present invention relates to a method for expansion culture of cartilage or bone progenitor cells, and a kit for the expansion culture.

### [Background Art]

Generally, human cartilage is not regenerated when it is congenitally deficient or in the case of acquired damage or defect. It is estimated that there are about 25 million people in Japan with knee osteoarthritis, which is mainly caused by the aging of articular cartilage, and the number is expected to increase further in the future with aging. Conventional treatments for such human cartilage diseases include a method of collecting cartilage tissue from other site of own body and transplanting the tissue to a defective site, but the collection site and the amount are limited. It is also difficult to proliferate chondrocytes derived from living body because chondrocytes have low proliferative potential and poor regenerative ability.

Therefore, a method of differentiating cells other than chondrocytes (e.g., pluripotent stem cells) into chondrocytes in vitro and returning them into the living body has been developed. As such method, for example, a method of inducing differentiation of pluripotent stem cells into chondrocytes has been reported (e.g., non-patent documents 1, 2, patent document 1, and the like). However, these methods require a period of at least 12 days to differentiate pluripotent stem cells into chondrocytes, and it is not possible to obtain chondrocytes in an amount sufficient for the treatment of cartilage diseases or bone diseases.

### [Document List]

### [Patent document]

patent document 1: WO 2016/141084

### [Non-patent documents]

non-patent document 1: Yamashita A., et al., Stem Cell Reports, 4(3): 404-418 (2015)
non-patent document 2: Loh K.M., et al., Cell, 166(2): 451-467 (2016)

### [Summary of Invention]

### [Technical Problem]

It is therefore an object of the present invention to provide a method capable of producing a chondrocyte or the like in an amount sufficient for use in the treatment of a disease, with a smaller number of days for differentiation into the chondrocyte or the like than by conventional methods.

### [Solution to Problem]

In an attempt to solve the above-mentioned problems, the present inventors changed the conventional idea of improving the differentiation efficiency from starting cells such as pluripotent stem cells and the like into chondrocytes by improving the type of cytokine or transcription factor and the timing of addition to the medium, and the like. They acquired an idea that, if progenitor cells of chondrocyte with high proliferative capacity can be identified, the induction time can be shortened and a large amount of chondrocytes can be obtained by proliferating the progenitor cells and inducing differentiation into chondrocytes. In embodying this idea, they made an assumption that a cartilage or bone progenitor cell having nearly infinite proliferative capacity (the present inventors named this cell as "iCOP (iPS-derived chondro/osteogenic progenitor)") exits as an intermediate cell in the process of differentiating induced pluripotent stem cells into chondrocytes. They actually identified iCOP and tried to establish a method for expansion culture of the cell. The present inventors first assumed that, among the cells contained in a cell population containing the germ-layer cell, a cell that shows proliferative capacity and can differentiate into chondrocyte in a short period of time is a sclerotome. Thus, they improved the method described in non-patent document 1 and induced differentiation of induced pluripotent stem cells into sclerotomes via mesodermal cells. As a result, they found that the cells highly express the markers described in non-patent document 1 such as PDGFRA, SOX9 and the like. Thereafter, they conducted intensive studies of the culture conditions under which sclerotomes can proliferate, and found that sclerotome can proliferate for at least 5 passages while maintaining expression of its cell markers (i.e., having potential for self-renewal) by culturing in a medium containing a TGF-β signal inhibitor and FGF. The above-mentioned finding was surprising because sclerotome was not known to have potential for self-renewal. Moreover, the sclerotome that was expansion cultured for such a long period of time still had potency to differentiate into chondrocytes. Therefore, it was concluded that sclerotome was the iCOP. The present inventors conducted further studies based on the above-mentioned finding, and completed the present invention.

Accordingly, the present invention relates to the following.
[1] A method for expansion culture of a cartilage or bone progenitor cell, comprising a step of culturing a cartilage or bone progenitor cell in a medium comprising a TGF-β signal inhibitor and FGF.
[2] The method of [1], wherein the aforementioned cartilage or bone progenitor cell is a cell that expresses PDGFRA, SOX9, or PAX1.
[3] The method of [1] or [2], wherein the aforementioned medium further comprises L-ascorbic acid or a derivative thereof.
[4] The method of any of [1] to [3], wherein the aforementioned TGF-β signal inhibitor is SB431542.
[5] The method of any of [1] to [4], wherein the aforementioned FGF is bFGF.
[6] The method of any of [1] to [5], wherein a concentration of the aforementioned TGF-β signal inhibitor in the medium is 1 µM - 50 µM.
[7] The method of any of [1] to [6], wherein a concentration of the aforementioned FGF in the medium is 1 ng/mL - 500 ng/mL.
[8] The method of any of [1] to [7], wherein the aforementioned cartilage or bone progenitor cell is derived from a pluripotent stem cell.
[9] The method of any of [1] to [8], wherein the aforementioned cartilage or bone progenitor cell is obtained by suspension culture of a pluripotent stem cell.
[10] The method of any of [1] to [9], wherein the expansion culture is suspension culture.
[11] A kit for expansion culture of a cartilage or bone progenitor cell, comprising a basal medium, a TGF-β signal inhibitor, and FGF.
[12] The kit of [11], further comprising L-ascorbic acid or a derivative thereof.
[13] The kit of [11] or [12], wherein the aforementioned TGF-β signal inhibitor is SB431542.
[14] The kit of any of [11] to [13], wherein the aforementioned FGF is bFGF.
[15] A method for producing a chondrocyte or osteocyte, comprising a step of inducing differentiation of a cartilage or bone progenitor cell obtained by the method of any of [1] to [10] into a chondrocyte or osteocyte.
[16] The method of [15], wherein the aforementioned differentiation induction step comprises a step of culturing the cartilage or bone progenitor cell in a medium comprising a BMP signal activator and/or a TGF-β signal activator.
[17] The method of [15] or [16], wherein the aforementioned TGF-β signal activator is TGF-β3.
[18] A method for producing a syndetome, comprising a step of inducing differentiation of a cartilage or bone progenitor cell obtained by the method of any of [1] to [10] into a syndetome.
[19] A method for producing a tendon cell or ligament cell, comprising a step of inducing differentiation of a syndetome obtained by the method of [18] into a tendon cell or ligament cell.
[20] An agent for cell transplantation therapy, comprising a cell obtained by the method of any of [15] to [19].
[21] A method for identifying a cartilage or bone progenitor cell, comprising a step of detecting or measuring expression of at least one gene selected from the group consisting of the following genes:
   A2M, ABCA9, ACAT2, ADAMTS9, ADGRF5, AGTR2, AMER1, AMOT, ANGPT1, ARHGAP5-AS1, ARHGEF26, ATP8A1, BCHE, BEGAIN, BOC, C3ORF52, CACNA1G, CELF2, CLSPN, COL25A1, COL26A1, CORO1A, CRISPLD1, CTTNBP2, CYB5B, DLGAP1, DNAJC12, DUSP9, EBF1, EBF2, EFEMP1, ESCO2, EYA1, FAM35A, FAM78A, FAR2, FGFBP2, FGFR4, FKBP4, FRZB, GCNT4, GNG11, HAAO, HMGCS1, HS3ST5, ID4, IGDCC3, IRF8, KCNA6, KCNB2, KITLG, LGR5, LHCGR, LHFP, LPPR5, LUM, MAPK8IP2, MECOM, MEF2C, MEOX2, METTL7A, NAB1, NCALD, NGF, NGFR, NKAIN2, NPR1, NR2F1, OLFML1, PCDH18, PCDH19, PCSK9, PDE4D, PDGFRA, PEG10, PLVAP, PRELP, PRRT4, RARRES2, RBMS3, RBPMS2, RSPO3, RUNX1T1, SAMD5, SCARA5, SKIDA1, SLC12A2, SLC27A3, SLC39A8, SLC7A2, SLC8A3, SLCO1C1, SOX8, SPRED2, STARD8, STOM, SYNPO, TBX18, TMC6, TNRC6C-AS1, TRIM2, TRIM9, TRIP13, TSPAN15, UBE2N, UHRF1, UNC5C, VIT, ZBTB46 and ZNF488.
[22] A method for isolating a cartilage or bone progenitor cell, comprising a step of isolating a cell that expresses at least one gene selected from the group consisting of the following genes from a cell population comprising the cartilage or bone progenitor cell:
   ABCA9, ATP8A1, BOC, KITLG, NKAIN2, ADGRF5, AGTR2, CACNA1G, C3ORF52, COL25A1, CYB5B, FAR2, FGFR4, GCNT4, HS3ST5, IGDCC3, LGR5, LHFP, LHCGR, NPR1, NGFR, PLVAP, PDGFRA, KCNA6, KCNB2, PCDH18, PCDH19, SCARA5, SLC12A2, SLC27A3, SLC39A8, SLC7A2, SLC8A3, SLCO1C1, STOM, TSPAN15, TMC6 and UNC5C.
[23] A reagent for isolating a cartilage or bone progenitor cell, comprising antibodies against each of proteins encoded by at least one gene selected from the group consisting of the following genes:
   ABCA9, ATP8A1, BOC, KITLG, NKAIN2, ADGRF5, AGTR2, CACNA1G, C3ORF52, COL25A1, CYB5B, FAR2, FGFR4, GCNT4, HS3ST5, IGDCC3, LGR5, LHFP, LHCGR, NPR1, NGFR, PLVAP, PDGFRA, KCNA6, KCNB2, PCDH18, PCDH19, SCARA5, SLC12A2, SLC27A3, SLC39A8, SLC7A2, SLC8A3, SLCO1C1, STOM, TSPAN15, TMC6 and UNC5C.

### [Advantageous Effects of Invention]

According to the present invention, expansion culture of a cartilage or bone progenitor cell such as the above-mentioned iCOP and the like becomes possible. The period for inducing differentiation from cartilage or bone progenitor cells into chondrocytes or osteocytes is half or less than the period for inducing differentiation from iPS cells into chondrocytes or osteocytes by a conventional method. Therefore, the period for inducing differentiation into chondrocytes or osteocytes can be drastically shortened by expansion culturing and stocking iCOP in advance. In addition, the proportion of undifferentiated cells such as pluripotent stem cells that can remain in the cell population before expansion culture relatively decreases by expansion culture of cartilage or bone progenitor cells, and the pluripotent stem cells are considered to differentiate into cartilage or bone progenitor cells and the like and decrease in number. Accordingly, the risk of undifferentiated cells being left in the cell population that cause tumor and the like can be remarkably reduced. In addition, an extremely large amount of chondrocytes or osteocytes can be produced by expansion culture of cartilage or bone progenitor cells during the differentiation process into chondrocytes as compared with the number of chondrocytes that can be produced by the above-mentioned conventional method. Therefore, chondrocytes in an amount sufficient for use in the treatment of cartilage diseases or bone diseases can be rapidly produced by the present invention.

### [Brief Description of Drawings]

Fig. 1 shows the results of the positive rate of SOX9 expressed by iCOP which was induced to differentiate from iPS cells, as measured by a flow cytometer. It was shown that iPS cells are differentiated into iCOP with high efficiency of about 90%.
Fig. 2 shows a proliferation curve when iCOP was proliferated for 5 passages every 7 days. It was shown from the proliferation curve that the population doubling (PD) per day was 0.46.
Fig. 3 shows the verification results of the influence of cryopreservation on the cell proliferative capacity of iCOP. It was shown that the cryopreservation does not decrease PD per day.
Fig. 4 shows the verification results of the relative expression level (mRNA amount) of iCOP marker genes in cryopreserved iCOP or not cryopreserved iCOP. It was shown that the cryopreservation does not decrease the relative expression level of iCOP marker genes.
Fig. 5 shows the evaluation results of relative expression level (mRNA amount) of osteocyte marker genes after inducing differentiation of iCOP into osteocytes. The iCOP was shown to maintain differentiation potential into osteocytes.
Fig. 6 shows the evaluation results of relative expression level (mRNA amount) of syndetome marker genes after inducing differentiation of iCOP into syndetomes. The iCOP was shown to maintain differentiation potential into ligament cells.
Fig. 7 shows a proliferation curve when iCOP was proliferated for 3 passages every 5 days by suspension culture. It was shown from the proliferation curve that the PD per day was 0.38.

### [Description of Embodiments]

### 1. Method of expansion culture of cartilage or bone progenitor cells

The present invention provides a method for expansion culture of cartilage or bone progenitor cells (hereinafter sometimes to be referred to as "the culture method of the present invention"). The culture method of the present invention includes a step of culturing cartilage or bone progenitor cells in a medium containing a TGF-β signal inhibitor and FGF.

In the present specification, the "expansion culture" means culture aiming to proliferate cartilage or bone progenitor cells contained in a cell population and increase the number of the cells. The increase in the cell number may be achieved when the number of increase by proliferation of the cells exceeds the number of decrease by death, and it does not require proliferation of all cells in the cell population.

In the present specification, the "cartilage or bone progenitor cell" means a cell having differentiation potential into chondrocyte and/or osteocyte. Preferably, cartilage or bone progenitor cells express PDGFRA, SOX9, or PAX1, more preferably PDGFRA, PAX1, and SOX9. Typically, the "cartilage or bone progenitor cell" is a sclerotome that expresses at least one member from PDGFRA, SOX9, and PAX1. When the cells differentiate into chondrocytes and/or osteocytes by at least one of the differentiation induction methods into chondrocytes and/or osteocytes described below, the cells can be evaluated as having the ability to differentiate into chondrocytes and/or osteocytes. Expression of PDGFRA, PAX1 and SOX9 in cells can be confirmed by at least one of real-time PCR and flow cytometry. In addition, cartilage or bone progenitor cells may have differentiation induction potency into cells other than cartilage or bone, such as syndetome. In the present specification, unless otherwise specified, the meaning of "expression" includes at least "production of a functional protein", preferably further includes "production of mRNA".

The "cartilage or bone progenitor cells" may express at least one, preferably two or more (e.g., 3, 4, 5, 6, 7, 8, 9, 10 or more), of the following marker genes, and such cells expressing at least PDGFRA gene are preferred.

A2M, ABCA9, ACAT2, ADAMTS9, ADGRF5, AGTR2, AMER1, AMOT, ANGPT1, ARHGAP5-AS1, ARHGEF26, ATP8A1, BCHE, BEGAIN, BOC, C3ORF52, CACNA1G, CELF2, CLSPN, COL25A1, COL26A1, CORO1A, CRISPLD1, CTTNBP2, CYB5B, DLGAP1, DNAJC12, DUSP9, EBF1, EBF2, EFEMP1, ESCO2, EYA1, FAM35A, FAM78A, FAR2, FGFBP2, FGFR4, FKBP4, FRZB, GCNT4, GNG11, HAAO, HMGCS1, HS3ST5, ID4, IGDCC3, IRF8, KCNA6, KCNB2, KITLG, LGR5, LHCGR, LHFP, LPPR5, LUM, MAPK8IP2, MECOM, MEF2C, MEOX2, METTL7A, NAB1, NCALD, NGF, NGFR, NKAIN2, NPR1, NR2F1, OLFML1, PCDH18, PCDH19, PCSK9, PDE4D, PDGFRA, PEG10, PLVAP, PRELP, PRRT4, RARRES2, RBMS3, RBPMS2, RSPO3, RUNX1T1, SAMD5, SCARA5, SKIDA1, SLC12A2, SLC27A3, SLC39A8, SLC7A2, SLC8A3, SLCO1C1, SOX8, SPRED2, STARD8, STOM, SYNPO, TBX18, TMC6, TNRC6C-AS1, TRIM2, TRIM9, TRIP13, TSPAN15, UBE2N, UHRF1, UNC5C, VIT, ZBTB46 and ZNF488.

The TGF-β signal inhibitor to be used in the present invention is not particularly limited as long as it is a substance that inhibits the signal transduction from the binding to the receptor of TGF-β to SMAD. For example, a substance that inhibits the binding to the ALK family, which is a receptor of TGF-β, a substance that inhibits phosphorylation of SMAD by the ALK family, and the like can be mentioned. Examples of the TGF-β signal inhibitor in the present invention include Lefty-1 (e.g., as NCBI Accession Nos, mouse: NM_010094, human: NM_020997), SB431542, SB202190 (both R.K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01 (WO 2009146408) and derivatives thereof, and the like. Among these, SB431542 is preferred.

The concentration of the TGF-β signal inhibitor in a medium is not particularly limited. When SB431542 is used, for example, 1 µM - 50 µM (e.g., 1 µM, 2 µM, 3 µM, 4 µM, 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, 20 µM, 25 µM, 30 µM, 35 µM, 40 µM, 45 µM, 50 µM) is preferred, though not limited to these. More preferably, it is 2 µM - 20 µM, particularly preferably 5 - 10 µM. When a TGF-β signal inhibitor other than SB431542 is used, the concentration can be set appropriately based on common technical knowledge.

The FGF to be used in the present invention is not particularly limited as long as it promotes proliferation of cells. For example, FGF-1, bFGF(FGF-2), FGF-3, FGF-4, FGF-5, FGF-6, FGF-7, FGF-8 (e.g., FGF-8b), FGF-9, FGF-10, FGF-11, FGF-12, FGF-13, FGF-14, FGF-15, FGF-16, FGF-17, FGF-18, FGF-19, FGF-20, FGF-21, FGF-22, FGF-23 and the like can be mentioned. Among these, bFGF is preferred. The above-mentioned FGF may be derived from any animal (e.g., rodents such as mouse, rat, hamster, guinea pig and the like, primates such as human, monkey, orangutan, chimpanzee and the like), and can be appropriately selected according to the type of the cells to be cultured. When a cell derived from human is culture, the FGF derived from human is preferably used. Specific Examples of the FGF include, but are not limited to, human bFGF (e.g., Endocrine Rev., 8, 95, 1987), bovine bFGF (e.g., Proc. Natl. Acad. Sci. USA, 81, 6963, 1984), mouse bFGF (e.g., Dev. Biol., 138, 454-463, 1990), rat bFGF (e.g., Biochem. Biophys. Res. Commun., 157, 256-263, 1988) and the like.

The FGF to be used in the present invention may be not only a natural form but also a variant form thereof. For example, as FGF variant, a chimeric protein in which a specific region of FGF1 (partial sequence at 41-83 positions or partial sequence at 62-83 positions of the amino acid sequence of human FGF1 protein) is replaced with a region of bFGF corresponding to the region is known to also have cell proliferation activity similar to that of bFGF (JP-A-2012-143234, JP-A-2014-100141). Thus, a variant of FGF containing the above-mentioned partial region of bFGF can also be preferably used in the present invention. The FGF to be used in the present invention can be produced by a method known per se, or can be obtained by purchasing a commercially available product, or the like. A medium containing FGF can also be produced by adding a soluble FGF to a medium, or by adding a carrier such as beads with FGF immobilized on the surface, and the like (e.g., StemBeads FGF2, etc.) to a medium.

The concentration of FGF in a medium is not particularly limited. When bFGF is used, for example, 1 ng/mL - 500 ng/mL (e.g., 1 ng/mL, 2 ng/mL, 3 ng/mL, 4 ng/mL, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, 15 ng/mL, 16 ng/mL, 17 ng/mL, 18 ng/mL, 19 ng/mL, 20 ng/mL, 25 ng/mL, 30 ng/mL, 35 ng/mL, 40 ng/mL, 45 ng/mL, 50 ng/mL, 100 ng/mL), though not limited to these. More preferably, it is 2 ng/mL - 100 ng/mL, particularly preferably 10 ng/mL. When an FGF other than bFGF is used, the concentration can be set appropriately based on common technical knowledge.

Examples of the basal medium to be used in the present invention include DMEM, EMEM, IMDM (Iscove's Modified Dulbecco's medium), GMEM (Glasgow's MEM), RPMI-1640, α-MEM, Ham's medium F-12, Ham's medium F-10, Ham's medium F12K, medium 199, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's MB752/1, CMRL-1066, Williams' medium E, Brinster's BMOC-3 medium, E8 medium (Nature Methods, 2011, 8, 424-429), ReproFF2 medium (ReproCELL Incorporated), a mixed medium of these (e.g., DMEM/F-12 mix medium, etc.), and the like.

The medium to be used in the present invention may contain additives known per se. Examples of such additive include growth factor (e.g., PDGF, insulin, etc.), iron source (e.g., transferrin, etc.), hedgehog signal activator, polyamines (e.g., putrescine, etc.), mineral (e.g., sodium selenate, etc.), saccharides (e.g., glucose, etc.), organic acid (e.g., pyruvic acid, lactic acid, etc.), serum protein (e.g., albumin, etc.), amino acid (e.g., L-glutamine, etc.), reducing agent (e.g., 2-mercaptoethanol, etc.), vitamins (e.g., vitamin Cs, d-biotin, etc.), steroid (e.g., β-estradiol, progesterone, etc.), antibiotic (e.g., streptomycin, penicillin, gentamicin, etc.), buffering agent (e.g., HEPES, etc.) and the like. Each additive is preferably contained within a concentration range known per se.

In the present specification, vitamin Cs mean L-ascorbic acid and a derivative thereof, and L-ascorbic acid derivative means one that becomes vitamin C by an enzymatic reaction in the living body. The L-ascorbic acid derivative to be used in the present invention is exemplified by vitamin C phosphate, ascorbyl glucoside, ascorbyl ethyl, vitamin C ester, ascorbyl tetraisopalmitate, ascorbyl stearate, ascorbic acid-2-phosphate-6 palmitate and the like. It is preferably vitamin C phosphate (e.g., Ascorbic acid 2-phosphate) and, for example, ascorbate L-phosphate such as sodium L-ascorbyl-phosphate, magnesium L-ascorbyl phosphate, and the like can be mentioned. The concentration of vitamin Cs in a medium is not particularly limited. When ascorbic acid 2-phosphate is used, its concentration in a medium is typically 20 µM - 2 mM (e.g., 50 µM, 100 µM, 150 µM, 200 µM, 250 µM, 300 µM, 500 µM, 1 mM, etc.). When vitamin C other than ascorbate 2-phosphate is used, the concentration can be set appropriately based on common technical knowledge.

The PDGF to be used in the present invention may be any of PDGF-AA, PDGF-AB, PDGF-BB, PDGF-CC, and PDGF-DD that bind to PDGF receptors, and PDGF-BB is preferred. The concentration of PDGF in a medium is not particularly limited. When PDGF-BB is used, its concentration in a medium is typically 10 ng/mL - 1 µg/mL (e.g., 50 ng/mL, 100 ng/mL, 150 ng/mL, 300 ng/mL, etc.). When PDGF other than PDGF-BB is used, the concentration can be set appropriately based on common technical knowledge.

The hedgehog signal activator to be used in the present invention is not particularly limited as long as it is a substance that activates a signal via a 12-transmembrane type patched (Ptc) and one of 7-transmembrane types, Smoothened (Smo). For example, hedgehog protein (e.g., Hh, Shh, Ihh, Dhh, etc.), Smoothened agonist (e.g., SAG (Hh-Ag 1.3), SAG21k (3-chloro-4,7-difluoro-N-(4-methoxy-3-(pyridin-4-yl)benzyl)-N-((1r,4r)-4-(methylamino)cyclohexyl)benzo[b]thiophene-2-carboxamide), Hh-Ag 1.1, Hh-Ag 1.5, purmorphamine and the like can be mentioned. Smoothened agonist is preferred, and SAG is more preferred. The concentration of the hedgehog signal activator in a medium is not particularly limited. When SAG is used, its concentration in a medium is typically 30 nM - 3 µM (e.g., 100 nM, 200 nM, 300 nM, 400 nM, 500 nM, 1 µM, etc.). When a hedgehog signal activator other than SAG is used, the concentration can be set appropriately based on common technical knowledge.

The medium to be used in the present invention may contain a serum. The serum is not particularly limited as long as it is derived from an animal, preferably a mammal (e.g., bovine embryo serum, human serum, etc.). The concentration of the serum only needs to be within the concentration range known per se. When cells proliferated by the culture method of the present invention, chondrocytes obtained by differentiation induction from the cells, and the like are used for medical purposes, it is preferable that a serum is not contained because xenogeneic components may become a source of blood-mediated pathogenic bacteria and heteroantigens. When a serum is not contained, an alternative additive of serum (e.g., Knockout Serum Replacement (KSR) (Invitrogen), Chemically-defined Lipid concentrated (Gibco), B-27 Supplement (Gibco), etc.) may also be used.

Examples of the incubator to be used in the present invention include flask, tissue culture flask, dish, petri dish, tissue culture dish, multidish, microplate, microwell plate, multiplate, multiwell plate, microslide, chamber slide, schale, tube, tray, culture bag, roller bottle and the like.

The incubator may be a cell-adhesive incubator used for adhesion culture, or a cell non-adhesive incubator used for suspension culture, and can be appropriately selected according to the purpose. A cell-adhesive incubator may be coated with any cell-supporting substrate such as extracellular matrix (ECM, also referred to as extracellular substrate) and the like for the purpose of improving the adhesiveness of the surface of the incubator to cells. The cell-supporting substrate may be any substance aiming at adhering cells. In the culture method of the present invention, when a feeder cell is not used, it is preferable to perform culture by using an extracellular substrate or an active fragment thereof, or an artificial material that mimics those functions. The extracellular substrate is not particularly limited as long as it is generally used for culturing cells for the purpose of improving adhesion between the surface of an incubator and cells. For example, known ones such as laminin (laminin 511, laminin 332, etc.), fibronectin, vitronectin, collagen, elastin, adhesamine and the like can be used. An active fragment of an extracellular substrate only needs to be a fragment having cell adhesion activity equivalent to that of the extracellular substrate, and known ones can be used. For example, E8 fragment of laminin 511 (e.g., iMatrix-511 (Nippi), etc.), E8 fragment of laminin 332, and the like disclosed in JP-A-2011-78370 can be mentioned. An extracellular substrate and an active fragment thereof may be commercially available products and are available from, for example, Life Technologies, BD Falcon, Biolamina, Nippi, and the like. Two or more kinds of these extracellular substrates and active fragments thereof may be used in combination. In addition, Matrigel (trade name) and Geltrex Matrix (trade name) may also be used, which are mixtures of complicated basement membrane components containing proteins and polysaccharides and extracted and purified from mouse EHS sarcoma that overproduces the basal lamina.

The extracellular substrate and active fragments thereof may be suspended in a suitable solution and applied to a container suitable for culturing cells. The artificial material that mimics the functions of an extracellular substrate is not particularly limited as long as it is generally used for culturing cells. For example, known ones such as Synthemax (registered trade mark) and Ultra-Web (registered trade mark) of Corning Incorporated, Hy-STEM series of Sigma-Aldrich Co. LLC, polylysine, polyornithine, and the like can be used. The extracellular substrate or an active fragment thereof, or an artificial material that mimics the functions thereof to be used in the present invention is preferably Matrigel, or laminin 511 or an active fragment of laminin 511, more preferably an active fragment of laminin 511 (i.e., E8 fragment of laminin 511).

According to the culture method of the present invention, a cartilage or bone progenitor cell can be endlessly proliferated (can be proliferated for at least 5 weeks). Therefore, the culture period is not particularly limited, and a period for achieving a desired number of cells can be appropriately selected. The culture temperature is not particularly limited and is 30 - 40°C, preferably 37°C. Culture is performed in the presence of CO₂-containing air, and the CO₂ concentration is preferably 2 - 5%.

As the cartilage or bone progenitor cell to be used in the present invention, for example, a cell isolated from a biological sample by cell sorting with PDGFRA as an index and the like may also be used, or a cell obtained by differentiation induction from a stem cell such as pluripotent stem cell, mesenchymal stem cell and the like may also be used. Preferably, it is a cell derived from a pluripotent stem cell.

In the present specification, the "pluripotent stem cell" means an embryonic stem cell (ES cell) and a cell having differentiation pluripotency similar to that of embryonic stem cell, namely, the ability to differentiate into various tissues of the body (all of endoderm, mesoderm, ectoderm). Examples of the pluripotent stem cell to be used in the present invention include embryonic stem cell (ES cell), induced pluripotent stem cell (iPS cell), pluripotent germ stem cell, embryonic germ cell (EG cell) and the like. It is preferably ES cell or iPS cell, and iPS cell is particularly preferred. When the above-mentioned pluripotent stem cell is any cell derived from ES cell or human embryo, the cell may be a cell produced by destroying an embryo or not destroying an embryo. It is preferably a cell produced by not destroying an embryo.

In the present specification, the "ES cell" means a pluripotent stem cell established from an inner cell mass of early embryo (e.g., blastocyst) of a mammal such as human, mouse and the like. ES cell was found in a mouse in 1981 (M.J. Evans and M.H. Kaufman (1981), Nature 292:154-156), and thereafter, an ES cell line was also established in primates such as human, monkey and the like (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165).

In the present specification, the "induced pluripotent stem cell" is a pluripotent stem cell induced from a somatic cell, and means a cell artificially conferred with pluripotency similar to that of embryonic stem cells by reprogramming somatic cells. For example, pluripotent iPS cells (induced pluripotent stem cells) established by reprogramming differentiated cells such as fibroblast and the like by expressing genes such as Oct3/4, Sox2, Klf4, Myc, and the like. In 2006, an induced pluripotent stem cell was established from mouse fibroblast by Yamanaka et. al. (Cell, 2006, 126(4), p663-676). In 2007, an induced pluripotent stem cell having multipotency similar to that of embryonic stem cells was established from human fibroblast (Cell, 2007, 131(5),p861-872; Science, 2007, 318(5858), p1917-1920; Nat Biotechnol., 2008, 26(1), p101-106).

As the origin of the pluripotent stem cells, cells derived from rodents such as mouse, rat, hamster, guinea pig and the like, and cells derived from primates such as human, monkey, orangutan, chimpanzee and the like can be mentioned, though not limited to these. A cell derived from human is preferred.

The pluripotent stem cell to be used in the present invention may be a cell established in advance and stocked, or may be a cell established newly. Therefore, a step of establishing a pluripotent stem cell may be performed prior to the culture method of the present invention. The step of establishing a pluripotent stem cell when the cell is an induced pluripotent stem cell is not particularly limited as long as it includes a step of introducing a specific reprogramming factor into the somatic cell. For example, a step of collecting somatic cells, introducing a reprogramming factor such as Oct3/4, Sox2, Klf4, Myc, or the like to allow for artificial expression, and then selecting and expansion culturing cells that have acquired pluripotency, a step of introducing reprogramming factors (Oct3/4, Sox2, Klf4, and c-Myc) into human peripheral blood lymphocytes by using a retrovirus vector or Sendaivirus vector and culturing them (Nishimura, T. et al. Cell Stem Cell 2013, 12, 114-126), and the like can be mentioned.

The somatic cell from which the induced pluripotent stem cell to be used in the present invention is derived is not particularly limited. Examples of the somatic cell include, but are not limited to, lymphocytes in the peripheral blood, fibroblast of the skin and the like, skin cell, visual cell, brain cell, hair cell, mouth mucosa, pneumocyte, hepatocyte, gastric mucosa cell, enterocyte, splenocyte, pancreatic cell, kidney cell, neural stem cell, hematopoietic stem cell, mesenchymal stem cell derived from wisdom tooth, and the like, tissue stem cell, tissue progenitor cell, blood cell (e.g., peripheral blood mononuclear cell (including T cell and non-T cell), cord blood cell, etc.), epithelial cell, endothelial cell (e.g., vascular endothelial cell), muscle cells, and the like.

Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, and the like. These reprogramming factors may be used alone or in combination. The combination of reprogramming factors is exemplified by the combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, Judson R.L. et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474:225-229.

In the present specification, the "mesenchymal stem cell" means stem cells derived from bone marrow or bone membrane, peripheral blood, umbilical cord blood, or adipose tissue and capable of differentiating into tissues of mesenchymal tissue system (adipose tissue, cartilage tissue, bone tissue, and the like). As such mesenchymal stem cells, bone marrow mesenchymal stem cells are preferred because they are easily collected from living tissues and a culture method after collection has been established. In addition, adipose tissue-derived mesenchymal stem cells are preferred because they are easily collected as redundant tissues from the living body and less invasive during collection.

The method for inducing differentiation of stem cells such as pluripotent stem cells, mesenchymal stem cells, and the like into cartilage or bone progenitor cells can be appropriately performed according to a method known per se. For example, as a method for inducing differentiation of pluripotent stem cells into cartilage or bone progenitor cells, a cell population during the process of differentiating pluripotent stem cells into chondrocytes (e.g., 4 - 8 days after induction of differentiation) can be used by selecting, as necessary, the cells expressing PDGFRA or the like, according to the methods described in patent document 1, non-patent documents 1, 2, JP-A-2005-511083, and the like. In addition, as a method for inducing differentiation of mesenchymal stem cells into cartilage or bone progenitor cells, for example, the method described in JP-A-2004-254655 and the like can be appropriately referred to.

More specifically, differentiation into cartilage or bone progenitor cells can be induced by, for example, (i) culturing pluripotent stem cells in a medium containing a Wnt signal activator, a BMP inhibitor, FGF and/or a TGF-β signal activator for about 1 - 2 days (preferably 1 day), (ii) culturing the cells in a medium containing a TGF-β signal inhibitor, a Wnt signal activator, a BMP inhibitor and/or FGF for about 1 - 2 days (preferably 1 day), (iii) culturing the cells in a medium containing a TGF-β signal inhibitor, a BMP inhibitor, a Wnt signal inhibitor and/or a MAPK/ERK kinase (MEK) inhibitor for about 1 - 2 days (preferably 1 day), and (iv) culturing the cells in a medium containing a Wnt signal inhibitor and/or a hedgehog signal activator for about 1 - 5 days (preferably 3 days).

Examples of the above-mentioned Wnt signal activator include CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile), WNT protein (e.g., Wnt-1, Wnt-2, Wnt-2b, Wnt-3a, Wnt-4, Wnt-5a, Wnt-5b, Wnt-6, Wnt-7a, Wnt-7a/b, Wnt-7b, Wnt-8a, Wnt-8b, Wnt-9a, Wnt-9b, Wnt-10a, Wnt-10b, Wnt-11, Wnt-16b, etc.), RSPO protein (e.g., RSP02), lithium chloride, TDZD8 (4-benzyl-2-methyl-1,2,4-thiadiazolidine-3,5-dione), BIO-acetoxime ((2'Z,3'E)-6-bromoindirubin-3'-acetoxime), A1070722 (1-(7-methoxyquinolin-4-yl)-3-[6-(trifluoromethyl)pyridin-2-yl]urea), HLY78 (4-ethyl-5-methyl-5,6-dihydro-[1,3]dioxolo[4,5-j]phenanthridine), CID 11210285 hydrochloride (2-amino-4-(3,4-(methylenedioxy)benzylamino)-6-(3-methoxyphenyl)pyrimidine hydrochloride), WAY-316606, (hetero) arylpyrimidine, IQ-1, QS-11, SB-216763, DCA, and the like, and CHIR99021 is preferred. When CHIR99021 is used, the concentration thereof in the medium is typically 0.5 µM - 100 µM (e.g., 5 µM, 10 µM, etc.).

Examples of the above-mentioned BMP inhibitor include NOGGIN, CHORDIN, LDN193189 (4-[6-(4-piperazin-1-yl-phenyl)-pyrazolo[1,5-a]pyrimidin-3-yl]-quinoline hydrochloride), DMH1 (4-[6-[4-(1-methylethoxy)phenyl]pyrazolo[1,5-a]pyrimidin-3-yl]-quinoline), Dorsomorphin (6-[4-[2-(1-piperidinyl)ethoxy]phenyl]-3-(4-pyridinyl)-pyrazolo[1,5-a]pyrimidine dihydrochloride), K02288 (3-[(6-amino-5-(3,4,5-trimethoxyphenyl)-3-pyridinyl]phenol), ML347 (5-[6-(4-methoxyphenyl)pyrazolo[1,5-a]pyrimidin-3-yl]quinoline), DMH-1 and the like, and LDN193189 is preferred. When LDN193189 is used, the concentration thereof in the medium is typically 0.03 µM - 3 µM (e.g., 0.3 µM, etc.).

As the above-mentioned FGF, for example, the same FGFs as those used in the aforementioned culture method of the present invention can be mentioned, and bFGF is preferred. When bFGF is used, the concentration thereof in the medium is typically 10 ng/mL - 1000 ng/mL (e.g., 100 ng/mL, etc.).

As the above-mentioned TGF-β signal activator, for example, TGF-β (e.g., TGF-β1, TGF-β2, TGF-β3), activin A, IDE1 (1-[2-[(2-carboxyphenyl)methylene]hydrazide]heptanoic acid), IDE2 (1-(2-cyclopentylidenehydrazide)-heptanedioic acid), Nodal and the like can be mentioned, and activin A is preferred. When activin A is used, the concentration thereof in the medium is typically 3 ng/mL - 300 ng/mL (e.g., 30 ng/mL, etc.).

As the above-mentioned TGF-β signal inhibitor, for example, the same signal inhibitors as those recited in the above-mentioned 1. can be mentioned, and SB431542 is preferred. When SB431542 is used, the concentration thereof in the medium is typically 1 µM - 100 µM (e.g., 10 µM, etc.).

Examples of the above-mentioned Wnt signal inhibitor include C59 (4-(2-methyl-4-pyridinyl)-N-[4-(3-pyridinyl)phenyl]benzeneacetamide), DKK1, IWP-2 (N-(6-methyl-2-benzothiazolyl)-2-[(3,4,6,7-tetrahydro-4-oxo-3-phenylthieno[3,2-d]pyrimidin-2-yl)thio]-acetamide), Ant1.4Br, Ant1.4CI, niclosamide, apicularen, bafilomycin, XAV939 (3,5,7,8-tetrahydro-2-[4-(trifluoromethyl)phenyl]-4H-thiopyrano[4,3-d]pyrimidin-4-one), IWR-1 (4-(1,3,3a,4,7,7a-hexahydro-1,3-dioxo-4,7-methano-2H-isoindol-2-yl)-N-8-quinolinyl-benzamide), NSC668036 (N-[(1,1-dimethylethoxy)carbonyl]-L-alanyl-(2S)-2-hydroxy-3-methylbutanoyl-L-alanine-(1S)-1-carboxy-2-methylpropyl ester hydrate), 2,4-diamino-quinazoline, quercetin, ICG-001 ((6S,9aS)-hexahydro-6-[(4-hydroxyphenyl)methyl]-8-(1-naphthalenylmethyl)-4,7-dioxo-N-(phenylmethyl)-2H-pyrazino[1,2-a]pyrimidine-1(6H)-carboxamide), PKF115-584, BML-284 (2-amino-4-[3,4-(methylenedioxy)benzylamino]-6-(3-methoxyphenyl)pyrimidine), FH-535, iCRT-14, JW-55, JW-67 and the like, and IWR-1 is preferred. When IWR-1 is used, the concentration thereof in the medium is typically 0.3 µM - 30 µM (e.g., 3 µM, etc.).

Examples of the above-mentioned MEK inhibitor include PD184352 (2-(2-chloro-4-iodophenylamino)-N-cyclopropylmethoxy-3,4-difluorobenzamide), PD98059 (2-(2-amino-3-methoxyphenyl)-4H-1-benzopyran-4-one), U0126, SL327, PD0325901 (N-[(2R)-2,3-Dihydroxypropoxy]-3,4-difluoro-2-[(2-fluoro-4-iodophenyl)amino]-benzamide), trametinib, cobimetinib, binimetinib and the like, and PD0325901 is preferred. When PD0325901 is used, the concentration thereof in the medium is typically 0.03 µM - 3 µM (e.g., 0.3 µM, etc.).

Examples of the above-mentioned hedgehog signal activator include hedgehog protein (e.g., Hh, Shh, Ihh, Dhh, etc.), Smoothened agonist (e.g., SAG (Hh-Ag 1.3), SAG21k (3-chloro-4,7-difluoro-N-(4-methoxy-3-(pyridin-4-yl)benzyl)-N-((1r,4r)-4-(methylamino)cyclohexyl)benzo[b]thiophene-2-carboxamide), Hh-Ag 1.1, Hh-Ag.1.5, purmorphamine and the like, and Smoothened agonist is preferred. Among them, SAG is more preferred. When SAG is used, the concentration thereof in the medium is typically 0.03 µM - 3 µM (e.g., 0.3 µM, etc.).

In a preferred embodiment, the medium used in the above-mentioned step (i) contains CHIR99021, LDN193189, bFGF and activin A, the medium used in the above-mentioned step (ii) contains SB431542, CHIR99021, LDN193189 and bFGF, and the medium used in the above-mentioned step (iii) contains SB431542, LDN193189, IWR-1 and PD0325901, and the medium used in the above-mentioned step (iv) contains IWR-1 and SAG.

The cartilage or bone progenitor cells thus induced to differentiate may be used as they are in the culture method of the present invention, or may be cryopreserved (to be also referred to as frozen storage), thawed before use, and then used in the culture method of the present invention. In addition, cartilage or bone progenitor cells after differentiation induction may be expansion cultured before cryopreservation. Therefore, in another embodiment of the present invention, a cryopreservation method of the cartilage or bone progenitor cells obtained by the culture method of the present invention, including a step of cryopreserving the cells, or a production method of a frozen stock produced by the method is provided. The cell freezing operation may be performed using a culture medium containing the cells immersed therein, a physiological buffer, and the like as a cryopreservation solution, and after adding a cryoprotective agent thereto, or a treatment of replacing the culture solution with a cryopreservation solution containing a cryoprotective agent, and the like. When the culture medium is replaced with a cryopreservation solution, the cryopreservation solution may be added after removing substantially all the culture medium, or the cryopreservation solution may be added while leaving a part of the culture medium. As the cryopreservation solution, a commercially available solution may be used, and examples thereof include STEM-CELLBANKER (registered trade mark) (ZENOAQ). The cells can be thawed by any known thawing method. For example, it is achieved by contacting the cryopreserved cells with a solid, liquid or gaseous medium (e.g., water, culture medium) at a temperature higher than the freezing temperature using a water bath, incubator, constant temperature reservoir, and the like.

The basal medium used for induction of differentiation from stem cells into cartilage or bone progenitor cells is not particularly limited, and the same basal medium as that used in the aforementioned culture method of the present invention can be mentioned. The medium may contain an additive, a serum and/or a serum replacement and, for example, the same additive, serum, and serum replacement as those that can be used in the aforementioned culture method of the present invention can be mentioned. In addition, an incubator used for the above-mentioned differentiation induction is not particularly limited, and the same incubator as that used in the aforementioned culture method of the present invention can be mentioned. The incubator may be cell adhesive or cell non-adhesive, and is appropriately selected according to the purpose. A cell-adhesive incubator may be coated with any cell-supporting substrate such as extracellular matrix (ECM, also referred to as extracellular substrate) and the like for the purpose of improving the adhesiveness of the surface of the incubator to cells. As such cell-supporting substrate, the same cell-supporting substrates as those that can be used in the aforementioned culture method of the present invention can be mentioned.

The cell culture method (including the culture method of the present invention) may be adhesion culture or suspension culture. In the present specification, the "suspension culture" means culturing target cells and cell aggregates without allowing them to adhere to the bottom surface of the incubator, and culturing in a state where the cells or cell aggregates are in contact with the bottom surface but they float in the culture medium when the culture medium is shaken lightly is also included in the suspension culture. The suspension culture may be a static culture. It can also be performed by a bioreactor (e.g., single-use bioreactor, etc.) or an automatic culture device which automatically performs cell seeding, medium exchange, cell image acquisition, and cultured cell recovery in a closed environment under mechanical control, and is capable of culturing at high density while controlling pH, temperature, oxygen concentration, and the like. As a method of supplying a new medium during culture using these devices and delivering required substances to cells and/or tissues in proper quantities, fed-batch culture, continuous culture and perfusion culture are available, and any method can be used in the present invention. The culture temperature is not particularly limited and is 30 - 40°C, preferably 37°C. Culture is performed in the presence of CO₂-containing air, and the CO₂ concentration is preferably 2 - 5%.

### 2. Kit for expansion culture of cartilage or bone progenitor cells

The present invention also provides a kit for expansion culture of cartilage or bone progenitor cells (hereinafter sometimes to be referred to as "the kit of the present invention"). The kit of the present invention contains a basal medium, a TGF-β signal inhibitor and FGF. As such basal medium, TGF-β signal inhibitor and FGF, for example, the same ones as those recited in the above-mentioned 1. can be mentioned. Among these, DMEM/F-12 mix medium is preferred as a basal medium, SB431542 is preferred as a TGF-β signal inhibitor, and bFGF is preferred as FGF.

The kit of the present invention may also contain a cartilage or bone progenitor cell, additive to medium, a serum, a serum replacement, an incubator and/or a cell-supporting substrate. As such cartilage or bone progenitor cell, additive to the medium, serum, serum replacement, incubator and cell-supporting substrate, for example, the same ones as those recited in the above-mentioned 1. can be mentioned. The kit of the present invention may further contain documents and instructions that describe the procedure for expansion culture.

The TGF-β signal inhibitor and/or FGF, and the above-mentioned additive to medium, serum, serum replacement, and the like as necessary to be contained in the kit of the present invention may be provided while being added to the basal medium in advance. Therefore, in one embodiment, a kit containing a medium containing a TGF-β signal inhibitor and/or FGF, or a medium for expansion culture of cartilage or bone progenitor cells, containing a TGF-β signal inhibitor and FGF is provided.

### 3. Production method of chondrocyte or osteocyte

The present invention also provides a production method of chondrocyte or osteocyte (hereinafter sometimes to be referred to as "the production method of the present invention"). The production method of the present invention includes a step of inducing differentiation of a cartilage or bone progenitor cell obtained by the culture method of the present invention (hereinafter sometimes to be referred to as "the progenitor cell of the present invention") into chondrocyte or osteocyte.

As a method for inducing differentiation of the progenitor cell of the present invention into chondrocyte, a method known per se can be used. For example, the progenitor cell of the present invention can be induced to differentiate into chondrocyte by appropriately referring to the methods described in patent document 1, non-patent documents 1, 2, JP-A-2005-511083, JP-A-2004-254655, and the like. More specifically, for example, differentiation into chondrocyte can be induced by culturing the progenitor cell of the present invention in a medium containing a BMP signal activator and/or a TGF-β signal activator.

Prior to starting differentiation induction (e.g., after cultivating cryopreserved cells), the progenitor cell of the present invention may be expansion cultured again by the method of the present invention, and specific methods and the basal medium, TGF-β signal inhibitor, FGF, medium additive, serum and alternative thereof, incubator and the like to be used are as described in the above-mentioned 1.

As the above-mentioned BMP signal activator, for example, bone morphogenic protein (BMP) (e.g., BMP-2, BMP-4, BMP-7, etc.), Alantolactone, FK506, isoliquiritigenin, 4'-hydroxychalcone and the like can be mentioned. Bone morphogenic proteins are preferred, and among them, BMP-4 is more preferred. When BMP-4 is used, the concentration thereof in the medium is typically 2 ng/mL - 200 ng/mL (e.g., 20 ng/mL, etc.). As the above-mentioned TGF-β signal inhibitor, for example, the same signal inhibitors as those recited in the above-mentioned 1. can be mentioned, and TGF-β is preferred and TGF-β3 is more preferred. When TGF-β3 is used, the concentration thereof in the medium is typically 1 ng/mL - 100 ng/mL (e.g., 10 ng/mL, etc.). In a preferred embodiment, the medium used for the above-mentioned culture contains BMP-4 and TGF-β3.

Confirmation of chondrocyte can be performed by confirming alcian blue staining and/or expression of one or more cartilage markers. Examples of the aforementioned cartilage marker include initial cartilage marker (e.g., COL2A1, etc.), cartilage marker (e.g., ACAN, EPIPHYCAN, etc.) and the like. In a preferred embodiment of the present invention, chondrocytes induced to differentiate from cartilage or bone progenitor cells express COL2A1, ACAN and EPIPHYCAN at at least mRNA levels. The expression of such markers can be detected by a method known per se, and the expression of marker proteins can be detected by immunological assays using antibodies such as ELISA method, immunostaining method, Westernblot method, flow cytometry. In addition, the expression of marker genes can be detected by nucleic acid amplification methods and/or nucleic acid detection methods such as real-time PCR, microarray, biochip RNAseq and the like.

As a method for inducing differentiation of the progenitor cell of the present invention into osteocyte, a method known per se can be used. For example, the progenitor cell of the present invention can be induced to differentiate into osteocyte by appropriately referring to the methods described in, for example, Mahmood A. et al., J. Bone Miner. Res., 25:1216-1233 (2010), Lee K.W. et al., Stem Cells Dev., 19:557-568 (2010), Hu J. et al., Tissue Eng. Part A 16:3507-3514 (2010), Zou L. et al., Sci. Rep., 3:2243 (2013), WO 2001/017562, and WO 2006/123699. More specifically, for example, differentiation into osteocyte can be induced by culturing the progenitor cell of the present invention in a medium containing dexamethasone, β-glycerophosphate and vitamin Cs, Rho kinase inhibitor and BMP signal activator, and/or calcium antagonist. The above-mentioned additives to the medium are each preferably contained within a concentration range known per se. Alternatively, a method of inducing differentiation of progenitor cell into chondrocyte and then differentiating the chondrocyte into osteocyte can also be used.

As the above-mentioned vitamin Cs, for example, the same ones as those recited in the above-mentioned 1. can be mentioned. Examples of the above-mentioned Rho kinase inhibitor include (+)-trans-4-(1-aminoethyl)-1-(4-puridylcarbamoyl)cyclohexane, (+)-trans-N-(1H-pyrrolo[2,3-b]pyridin-4-yl)-4-(1-aminoethyl)cyclohexanecarboxamide, (R)-(+)-N-(4-pyridyl)-4-(1-aminoethyl)benzamide, (R)-(+)-N-(1H-pyrrolo[2,3-b]pyridin-4-yl)-4-(1-aminoethyl)benzamide and the like. As the above-mentioned BMP signal activator, for example, the same ones as those recited above can be mentioned. Bone morphogenic proteins are preferred, and BMP-2 is more preferred among them. Examples of the above-mentioned calcium antagonist include dihydropyridine calcium antagonist (e.g., benidipine, nifedipine, amlodipine, cilnidipine, etc.), phenylalkylamine calcium antagonist (e.g., verapamil, gallopamil, bepridil, etc.), benzothiazepine calcium antagonist (e.g., diltiazem), zonisamide, fasudil, lomerizine, pregabalin, cyclandelate, idebenone, buflomedil, atosiban and the like.

Confirmation of osteocyte can be performed by confirming alkali phosphatase activity and/or expression of one or more bone markers. Examples of the aforementioned bone marker include RUNX2, COL1A1, Osteopontin (OPN), Osterix, ALP, Osteocalcin and the like. In a preferred embodiment of the present invention, osteocytes induced to differentiate from cartilage or bone progenitor cells express COL2A1 and OPN at at least mRNA levels. Confirmation of alkali phosphatase activity can be performed using a method known per se (e.g., Kind-King method, Bessey-Lowry method, GSCC method, SSCC method, JSCC method, etc.) or a commercially available kit (e.g., TRACP & ALP Assay Kit (Takara Bio), etc.). The expression of bone markers can be detected by a method similar to that for the aforementioned detection of the expression of cartilage markers.

The basal medium used for induction of differentiation from the progenitor cell of the present invention into chondrocyte or osteocyte is not particularly limited, and the same basal medium as that used in the aforementioned culture method of the present invention can be mentioned. The medium may contain an additive, a serum and/or a serum replacement and, for example, the same additive, serum, and serum replacement as those that can be used in the aforementioned culture method of the present invention can be mentioned. In addition, an incubator used for the above-mentioned differentiation induction is not particularly limited, and the same incubator as that used in the aforementioned culture method of the present invention can be mentioned. The incubator may be cell adhesive or cell non-adhesive, and is appropriately selected according to the purpose. A cell-adhesive incubator may be coated with any cell-supporting substrate such as extracellular matrix (ECM, also referred to as extracellular substrate) and the like for the purpose of improving the adhesiveness of the surface of the incubator to cells. As such cell-supporting substrate, the same cell-supporting substrates as those that can be used in the aforementioned culture method of the present invention can be mentioned.

When the progenitor cell of the present invention is induced to differentiate into chondrocyte, the culture period is not particularly limited as long as chondrocyte is obtained by differentiation, and 3 days or more (e.g., 4 days, 5 days or more) is preferred. The upper limit is not particularly set, and 20 days or less (e.g., 15 days, 10 days, 9 days, 8 days, 7 days or less) is preferred. In a preferred embodiment, it is 6 days. When the progenitor cell of the present invention is induced to differentiate into osteocyte, the culture period is not particularly limited as long as osteocyte is obtained by differentiation, and 7 days or longer (e.g., 8 days, 9 days, 10 days, 11 days, 12 days, 13 days or longer) is preferred. The upper limit is not particularly set, and 30 days or less (e.g., 25 days, 20 days, 19 days, 18 days, 17 days, 16 days, 15 days or less) is preferred and, for example, 14 days can be mentioned. The culture temperature is not particularly limited and is 30 - 40°C, preferably 37°C. Culture is performed in the presence of CO₂-containing air, and the CO₂ concentration is preferably 2 - 5%.

The culture of the progenitor cell of the present invention may be an adhesion culture or suspension culture. The suspension culture may be a static culture, or can also be performed using the aforementioned bioreactor or automatic culture device. For culture using these devices, any of the methods of fed-batch culture, continuous culture and perfusion culture can be used in the present invention. The culture temperature is not particularly limited and is 30 - 40°C, preferably 37°C. Culture is performed in the presence of CO₂-containing air, and the CO₂ concentration is preferably 2 - 5%.

### 4. Production method of syndetome

Furthermore, the present invention provides a method for producing syndetome (hereinafter sometimes to be referred to as "the production method of syndetome of the present invention"). The production method of syndetome of the present invention includes a step of inducing differentiation of the progenitor cell of the present invention into syndetome. In the following, syndetome produced in this way is sometimes referred to as "the syndetome of the present invention". A tendon cell or a ligament cell can also be produced by inducing differentiation of the syndetome of the present invention into a tendon cell or a ligament cell. In the present specification, the term "sclerotome-lineage cell of the present invention" may be used to encompass chondrocyte, osteocyte, syndetome, tendon cell, ligament cell, and the progenitor cell of the present invention which are produced as mentioned above.

As a method for inducing differentiation of the progenitor cell of the present invention into syndetome, a method known per se can be used. For example, the progenitor cell of the present invention can be induced to differentiate into syndetome by appropriately referring to the methods described in Nakajima T. et al., Development, 145(16): dev165431 (2018), and the like. More specifically, for example, differentiation into syndetome can be induced by (A) a step of culturing the progenitor cell of the present invention in a medium containing FGF and/or TGF-β signal activator, and (B) culturing the cells cultured in the aforementioned step (A) in a medium containing BMP signal activator and/or TGF-β signal activator. The medium used in steps (A) and (B) preferably contains vitamin Cs. Specific examples of vitamin C include the same vitamin Cs as those recited in the above-mentioned 1. The concentration of vitamin Cs in the medium is also preferably similar to that shown in the above-mentioned 1.

As the above-mentioned FGF, the same FGFs as those recited in the above-mentioned 1. can be mentioned, and FGF-8 (e.g., FGF-8b) is preferred. When FGF-8 is used, the concentration thereof in the medium is typically 1 ng/mL - 100 ng/mL (e.g., 10 ng/mL, etc.).

As the above-mentioned TGF-β signal activator, for example, the same signal activators as those recited in the above-mentioned 1. can be mentioned, and TGF-β is preferred and TGF-β3 is more preferred among them. When TGF-β3 is used, the concentration thereof in the medium is typically 1 ng/mL - 100 ng/mL (e.g., 10 ng/mL, etc.).

As the above-mentioned BMP signal activator, the same signal activators as those recited in the above-mentioned 3. can be mentioned, and BMP-4 is preferred. When BMP-4 is used, the concentration thereof in the medium is typically 1 ng/mL - 100 ng/mL (e.g., 10 ng/mL, etc.).

In a preferred embodiment, when used in the above-mentioned step (A), TGF-β3 and FGF-8 are contained, and/or the medium used in the above-mentioned step (B) contains BMP-4 and TGF-β3.

Confirmation of syndetome can be performed by confirming expression of two or more syndetome markers. When expression of syndetome marker is confirmed, the cells may be cultured while applying a mechanical stress. As the aforementioned syndetome marker, two or more markers selected from, for example, COL1A1, TNMD and SCX, and the like can be mentioned. In a preferred embodiment of the present invention, syndetomes induced to differentiate from cartilage or bone progenitor cells express COL2A1 and TNMD at at least mRNA levels. The expression of such markers can be detected by a method known per se, and the expression of marker proteins can be detected by immunological assays using antibodies such as ELISA method, immunostaining method, Westernblot method, flow cytometry. In addition, the expression of marker genes can be detected by nucleic acid amplification methods and/or nucleic acid detection methods such as real-time PCR, microarray, biochipRNAseq and the like.

As a method for inducing differentiation of the syndetome of the present invention into tendon cell or ligament cell, a method known per se can be used. For example, the syndetome of the present invention can be induced to differentiate into tendon cell or ligament cell by appropriately referring to the method described in, for example, JP-A-2011-205964, and the like.

The basal medium used for induction of differentiation into syndetome, tendon cell or ligament cell is not particularly limited, and the same basal medium as that used in the aforementioned culture method of the present invention can be mentioned. The medium may contain an additive, a serum and/or a serum replacement and, for example, the same additive, serum, and serum replacement as those that can be used in the aforementioned culture method of the present invention can be mentioned. In addition, an incubator used for the above-mentioned differentiation induction is not particularly limited, and the same incubator as that used in the aforementioned culture method of the present invention can be mentioned. The incubator may be cell adhesive or cell non-adhesive, and is appropriately selected according to the purpose. A cell-adhesive incubator may be coated with any cell-supporting substrate such as extracellular matrix (ECM, also referred to as extracellular substrate) and the like for the purpose of improving the adhesiveness of the surface of the incubator to cells. As such cell-supporting substrate, the same cell-supporting substrates as those that can be used in the aforementioned culture method of the present invention can be mentioned.

When the progenitor cell of the present invention is differentiated into syndetome, the culture period is not particularly limited as long as syndetome is obtained by differentiation, and 3 days or more (e.g., 4 days, 5 days or more) is preferred. The upper limit is not particularly set, and 20 days or less (e.g., 15 days, 10 days, 9 days, 8 days, 7 days or less) is preferred. In a preferred embodiment, it is 8 days. The culture period in step (A) is preferably 1 - 3 days (e.g., 2 days), and the period of step (B) is preferably 4 - 8 days (e.g., 6 days). Also, the culture period when syndetome is differentiated into a tendon cell or ligament cell is not particularly limited as long as the differentiation into a tendon cell or ligament cell can be performed. The culture temperature is not particularly limited and is 30 - 40°C, preferably 37°C. Culture is performed in the presence of CO₂-containing air, and the CO₂ concentration is preferably 2 - 5%. The aforementioned culture may be an adhesion culture or suspension culture. The suspension culture can be performed in the same manner as the method described in the above-mentioned 1.

### 5. Cell transplantation therapy agent

The present invention also provides an agent for cell transplantation therapy, containing the sclerotome-lineage cell of the present invention (hereinafter to be also referred to as "cell transplantation therapy agent of the present invention"), and a production method of the agent. As described above, cartilage or bone progenitor cells in an amount sufficient for treating or preventing cartilage diseases or bone diseases can be obtained by the culture method of the present invention. In addition, by inducing differentiation of the cells, chondrocytes or osteocytes in an amount sufficient for treating or preventing cartilage diseases or bone diseases can be provided. Similarly, a syndetome, tendon cell or ligament cell obtained by inducing differentiation from the progenitor cell of the present invention can be used for the treatment or prophylaxis of tendon or ligament-related diseases. In addition, according to the culture method of the present invention, a cell population with a remarkably reduced risk of undifferentiated cells being left therein that cause tumors and the like can be obtained. Therefore, the sclerotome-lineage cell of the present invention is suitable for use as a starting material for a cell transplantation therapy agent, and the cell transplantation therapy agent is useful for the treatment or prophylaxis of a cartilage disease or bone disease such as osteoarthritis, rheumatoid arthritis, and the like or a tendon or ligament related disease such as tendon damage, Ehlers-Danlos syndrome, and the like. In the present specification, the sclerotome-lineage cell of the present invention also encompasses a cell population containing the cell.

Therefore, in one embodiment of the present invention, a method for producing a cell transplantation therapy agent of the present invention, including (1) a step of providing a cartilage or bone progenitor cell by the culture method of the present invention, and (2) a step of preparing a preparation containing an effective amount of the progenitor cell of the present invention provided in step (1) is provided. In another embodiment, a method for producing a cell transplantation therapy agent of the present invention, including (1') a step of providing chondrocyte or osteocyte by the production method of the present invention, and (2') a step of preparing a preparation containing an effective amount of the chondrocyte of the present invention provided in step (1') is provided. A preparation containing syndetome, tendon cell or ligament cell can also be prepared in the same manner as described above. In another embodiment, a method for treating or preventing a cartilage disease or a bone disease, including administering or transplanting an effective amount of the sclerotome-lineage cell of the present invention or the cell transplantation therapy agent of the present invention to a mammal (e.g., human, mouse, rat, monkey, bovine, horse, swine, dog, etc.) as the subject of treatment or prophylaxis is provided.

When the sclerotome-lineage cell of the present invention is used for a cell transplantation therapy agent, it is desirable to use a cell derived from an iPS cell established from a somatic cell having the same or substantially the same HLA genotype as that of an individual who receives the transplantation, because the rejection does not occur. As used herein, being "substantially the same" means that the HLA genotype of the transplanted cells matches to the extent that an immune response to the transplanted cells can be suppressed by an immunosuppressant. For example, a somatic cell having an HLA type in which three loci of HLA-A, HLA-B and HLA-DR or four loci with HLA-C added thereto match can be mentioned. When the sclerotome-lineage cell of the present invention has a gene mutation that causes cartilage disease, bone disease, and the like, for example, it is preferable to repair the gene mutation that causes the diseases in advance by using techniques such as genome editing (e.g., CRISPR system, TALEN, ZFN, etc.) and the like. When sufficient cells cannot be obtained due to age, physical constitution, and the like it is possible to implant the cells in a capsule such as polyethylene glycol or silicon, a porous container, and the like to avoid rejection.

The sclerotome-lineage cell of the present invention is produced as a parenteral preparation such as injection, suspension, drip transfusion and the like by mixing with a pharmaceutically acceptable carrier, and the like by a conventional means. Examples of the pharmaceutically acceptable carrier that can be contained in the parenteral preparation include saline, aqueous solution for injection such as an isotonic solution containing glucose and other auxiliary agents (e.g., D-sorbitol, D-mannitol, sodium chloride, and the like), and the like. The cell transplantation therapy agent of the present invention may be blended with, for example, buffering agent (e.g., phosphate buffer, sodium acetate buffer), soothing agent (e.g., benzalkonium chloride, procaine hydrochloride, and the like), stabilizer (e.g., human serum albumin, polyethylene glycol, and the like), preservative, antioxidant and the like. When the transplantation therapy agent of the present invention is formulated as an aqueous suspension, the sclerotome-lineage cells of the present invention are suspended in the above-mentioned aqueous solution at about 1×10⁶ - about 1×10⁸ cells/mL. In addition, the dose or transplantation amount, the number of administrations or the number of transplantations of the sclerotome-lineage cells or cell transplantation therapy agent of the present invention can be appropriately determined based on the age, body weight, condition, and the like of a mammal that receives the administration.

The cell transplantation therapy agent of the present invention can also be provided in a cryopreserved state under the conditions generally used for cryopreservation of cells, and thawed before use. In that case, it may further contain serum or a substitute thereof, an organic solvent (e.g., DMSO) and the like. In this case, the concentration of the serum or a substitute thereof is not particularly limited, and may be about 1 - about 30%(v/v), preferably about 5 - about 20%(v/v). The concentration of the organic solvent is not particularly limited, and may be 0 - about 50%(v/v), preferably about 5 - about 20%(v/v).

### 5. Other use

Furthermore, the sclerotome-lineage cell of the present invention is useful for screening for a compound for the treatment or prophylaxis of cartilage diseases or bone diseases, or tendon or ligament related diseases. For example, when a test compound, alone or in combination with other medicament, is contacted with the sclerotome-lineage cell of the present invention and, by a method known per se, the differentiation, proliferation or maturation of the cell is promoted, or the production amount of a cartilage substrate or bone substrate increases, the test compound can be evaluated to be useful as a compound for treating the above-mentioned diseases. The cell to be used for the above-mentioned screening is preferably a cell showing a phenotype similar to that of the disease to be treated, particularly preferably a cell produced by differentiation induction of an induced pluripotent stem cell produced from a somatic cell derived from the diseased patient. As the above-mentioned cartilage disease or bone disease, the same diseases as those recited in the above-mentioned 4. can be mentioned.

Examples of the test compound include peptide, protein, antibody, non-peptide compound, synthetic compound, fermented product, cell extract, plant extract, animal tissue extract, plasma and the like. The test compound here may form a salt. As the salt, a salt with a physiologically acceptable acid (e.g., inorganic acid, organic acid), a base (e.g., alkali metal salt, alkaline earth metal salt, aluminum salt), or the like is used. As such salt, a salt with an inorganic acid (e.g., hydrochloric acid, phosphoric acid, hydrobromic acid, sulfuric acid), a salt with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), a sodium salt, a potassium salt, a calcium salt, a magnesium salt, a barium salt, or an aluminum salt is used.

The sclerotome-lineage cell of the present invention can also be used further for the verification of a drug discovery target, analysis of disease mechanism, and the like.

### 6. Identification method of cartilage or bone progenitor cells

As shown in the Examples below, it was demonstrated that a cartilage or bone progenitor cell, iCOP, expresses the following genes. Therefore, a cartilage or bone progenitor cell can be identified by detecting or measuring expression of at least one gene selected from the group consisting of the following genes. In this method, a cell in which expression of at least one, preferably two or more (e.g., 3, 4, 5, 6, 7, 8, 9, 10 or more), of the following genes has been detected can be identified as a cartilage or bone progenitor cell, and it is preferable that the expression of at least PDGFRA gene is detected. The expression of genes can be detected or measured by a method similar to that for the aforementioned detection or measurement of the expression of cartilage markers.

A2M, ABCA9, ACAT2, ADAMTS9, ADGRF5, AGTR2, AMER1, AMOT, ANGPT1, ARHGAP5-AS1, ARHGEF26, ATP8A1, BCHE, BEGAIN, BOC, C3ORF52, CACNA1G, CELF2, CLSPN, COL25A1, COL26A1, CORO1A, CRISPLD1, CTTNBP2, CYB5B, DLGAP1, DNAJC12, DUSP9, EBF1, EBF2, EFEMP1, ESCO2, EYA1, FAM35A, FAM78A, FAR2, FGFBP2, FGFR4, FKBP4, FRZB, GCNT4, GNG11, HAAO, HMGCS1, HS3ST5, ID4, IGDCC3, IRF8, KCNA6, KCNB2, KITLG, LGR5, LHCGR, LHFP, LPPR5, LUM, MAPK8IP2, MECOM, MEF2C, MEOX2, METTL7A, NAB1, NCALD, NGF, NGFR, NKAIN2, NPR1, NR2F1, OLFML1, PCDH18, PCDH19, PCSK9, PDE4D, PDGFRA, PEG10, PLVAP, PRELP, PRRT4, RARRES2, RBMS3, RBPMS2, RSPO3, RUNX1T1, SAMD5, SCARA5, SKIDA1, SLC12A2, SLC27A3, SLC39A8, SLC7A2, SLC8A3, SLCO1C1, SOX8, SPRED2, STARD8, STOM, SYNPO, TBX18, TMC6, TNRC6C-AS1, TRIM2, TRIM9, TRIP13, TSPAN15, UBE2N, UHRF1, UNC5C, VIT, ZBTB46 and ZNF488.

### 7. Isolation method of cartilage or bone progenitor cells

A cartilage or bone progenitor cell can be isolated from a cell population containing cartilage or bone progenitor cells, by using, as an index, an antigen encoded by at least one, preferably two or more (e.g., 3, 4, 5, 6, 7, 8, 9, 10 or more), genes selected from the following cell surface antigen genes (hereinafter sometimes to be referred to as " the cell surface antigen gene of the present invention", and an antigen encoded by the gene is sometimes to be referred to as "the cell surface antigen of the present invention"), from among the above-mentioned proteins. As one of the cell surface antigens of the present invention, PDGFRA is preferred. The cell can be isolated by a method known per se (e.g., FACS, MACS, etc.) can be performed.

ABCA9, ATP8A1, BOC, KITLG, NKAIN2, ADGRF5, AGTR2, CACNA1G, C3ORF52, COL25A1, CYB5B, FAR2, FGFR4, GCNT4, HS3ST5, IGDCC3, LGR5, LHFP, LHCGR, NPR1, NGFR, PLVAP, PDGFRA, KCNA6, KCNB2, PCDH18, PCDH19, SCARA5, SLC12A2, SLC27A3, SLC39A8, SLC7A2, SLC8A3, SLCO1C1, STOM, TSPAN15, TMC6 and UNC5C.

### 8. Reagent for isolation of cartilage or bone progenitor cells

The present invention also provides a reagent for isolating a cartilage or bone progenitor cell, containing at least an antibody to each of one or more antigens from the cell surface antigens of the present invention (hereinafter sometimes to be referred to as "the reagent of the present invention"). When the reagent of the present invention contains antibodies to two or more antigens, the reagent may be provided as a reagent kit containing each antibody in a separate reagent. The antibody contained in the reagent of the present invention can be provided, for example, in a form bound to a fluorescent dye, metal isotope or bead (e.g., magnetic bead), depending on the isolation means. In the present specification, the antibody also includes antibody fragments and variants (e.g., Fab fragment, scFab fragment, ScFv fragment, etc.) that have the ability to bind to the cell surface antigens.

### [Example]

The present invention is explained in detail in the following by referring to Examples. However, the present invention is not limited to them.

### Example 1: Production of iCOP having proliferative capacity and cartilage differentiation potential

In the following Examples, iCOP (iPS cell-induced chondro/osteogenic progenitor) was produced from induced pluripotent stem cells (iPS cells), and its proliferative potential and differentiation potential into chondrocytes were evaluated. As the iPS cell, the 1210B2 strain purchased from iPS Academia Japan was used.

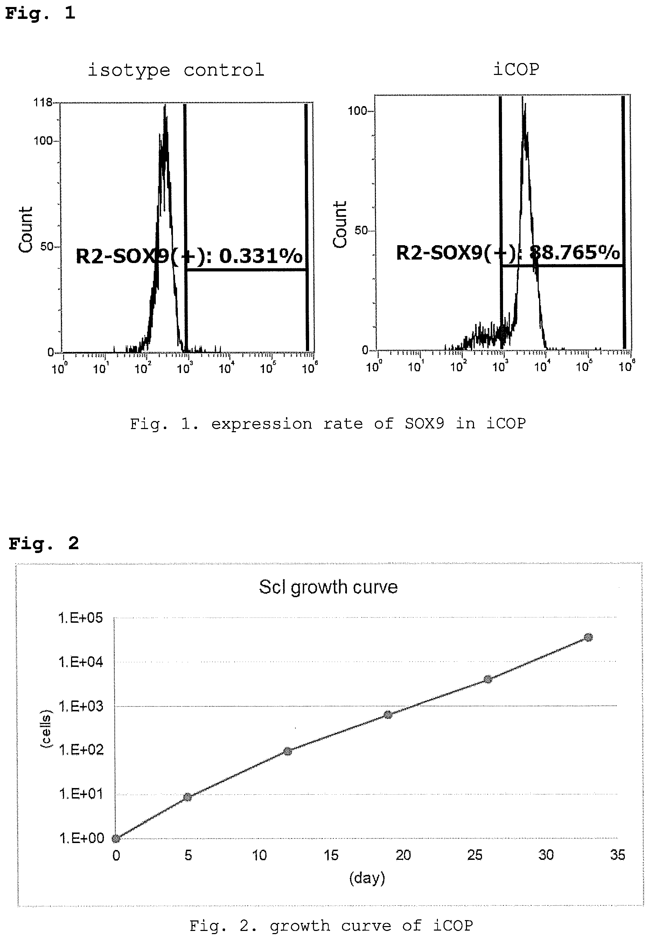

Using a 5 mL single-use bioreactor (Biott), iPS cells were seeded at a cell density of 2×10⁵ cells/mL to StemFit (registered trade mark) AK03N (Ajinomoto Co., Inc.) containing 10 µM Y-27632 (Wako) two days prior to the start of differentiation, and cultured with stirring in an incubator at 37°C, 5%CO₂ at a stirring rate of 80 rpm. Vi-CELLL (registered trade mark) XR (Beckman Coulter) was used to measure the number of viable cells. Thereafter, the medium was replaced with 5 mL of differentiation 1 medium containing RPMI1640 (Nacalai Tesque), 2% B27 (Thermo Fisher Scientific), 30 ng/mL Activin A (Ajinomoto Co., Inc.), 10 µM CHIR99021 (Miltenyi Biotech), 100 ng/mL bFGF (PeproTech), 0.3 µM LDN193189 (Wako). On the first day of differentiation, the medium was replaced with 5 mL of differentiation 2 medium containing RPMI1640, 2% B27, 10 µM SB431542 (ReproCell), 5 µM CHIR99021, 100 ng/mL bFGF, 0.3 µM LDN193189. On day 2, the medium was replaced with 5 mL of differentiation 3 medium containing RPMI1640, 2% B27, 10 µM SB431542, 3 µM IWR-1 (Sigma), 0.3 µM PD0325901 (Cayman Chemical), 0.3 µM LDN193189. On day 3, the medium was replaced with 5 mL of differentiation 4 medium containing RPMI1640, 2% B27, 3 µM IWR-1, 0.3 µM SAG (Cayman Chemical). On days 4 and 5, 70% of the culture supernatant of the single-use bioreactor was exchanged with differentiation 4 medium, and the cells on day 6 were used as iCOP. Then, SOX9 expressed on iCOP was labeled with Alexa Fluor (registered trade mark) 647 Mouse Anti-Sox9 (BD Biosciences), and the positive rate thereof was measured by Attune NxT Flow Cytometer (Thermo Fisher Scientific). As a result, it was confirmed that iCOP can be induced with high efficiency of about 90% (Fig. 1).

To evaluate the proliferative capacity of iCOP, iCOP was harvested from a single-use bioreactor, centrifuged, suspended in 1 mL of Accumax cell detachment solution (Merck), and allowed to stand for 10 min. The cells were suspended again until the cell aggregates were broken, centrifuged, suspended in iCOP proliferation medium containing DMEM/F12 (Thermo Fisher Scientific), 2% B27, 5 µM SB431542, 10 ng/mL bFGF, 250 µM L-ascorbic acid-2-phosphoric acid sesquimagnesium salt (Sigma), and the number of cells was counted. 2×10⁵ cells of iCOP were seeded on a 100 mm dish containing 8 mL of iCOP proliferation medium and incubated under the conditions of 37°C, 5% CO₂. After 3 and 5 days from seeding, the medium was replaced with 8 mL of a fresh medium and the cells were passaged every 7 days. In the Figures and Tables below, P0 shows no passage, P1 shows after one passage, P2 shows after two passages, P3 shows after three passages, P4 shows after four passages, and P5 shows after five passages. Five passages were repeated and the growth curve of iCOP was determined. As a result, the daily population doubling (PD) was 0.46 (Fig. 2). In addition, the gene expression of iCOP markers (PAX1, SOX9, PDGFRA) was measured to find that the expression was maintained even after 5 passages (Table 1).

### [Table 1]

**Table 1. gene expression of iCOP markers**

| | iCOP | | | |
|---|---|---|---|---|
| | P0 | P1 | P3 | P5 |
| *PAX1* | + | + | + | + |
| *SOX9* | + | + | + | + |
| *PDGFRA* | + | + | + | + |

### Example 2: Verification of cartilage differentiation potential of iCOP

To verify the cartilage differentiation potential of iCOP, iCOP at each passage number was washed with PBS(-), immersed in Accutase (Nacalai Tesque) and incubated at 37°C for 5 min. When the cells were detached, they were harvested in a tube, centrifuged, suspended in differentiation 5 medium containing DMEM/F12, 2% B27, 10 ng/mL TGF-β3 (PeproTech), 20 ng/mL BMP4 (R&D systems), and the number of cells was counted. 1×10⁶ cells of iCOP were seeded on an Elplasia^{™} Non-adherent surface 24 well plate (Kuraray) containing 1 mL of differentiation 5 medium, and incubated under the conditions of 37°C, 5% CO₂. Half the amount of the culture supernatant was exchanged every day, and the cells were harvested on day 6. The gene expression of chondrocytes after culture was evaluated by the real-time PCR method. As a result, even after 5 passages, iCOP maintained the differentiation potential into chondrocytes (Table 2).

### [Table 2]

**Table 2. gene expression of chondrocyte induced from iCOP**

| | chondrocyte | | | | |
|---|---|---|---|---|---|
| | P0 | P1 | P2 | P4 | P5 |
| *COL2A1* | + | + | + | + | + |
| *ACAN* | + | + | + | + | + |
| *EPIPHYCAN* | + | + | + | + | + |

### Example 3: Verification of cryopreservability of iCOP

In the following Experimental Example, iCOP produced from iPS cells was cryopreserved and thereafter thawed, and its proliferative capacity and differentiation potential into chondrocytes were evaluated. As the iPS cell, the 1231A3 strain purchased from iPS Academia Japan was used.

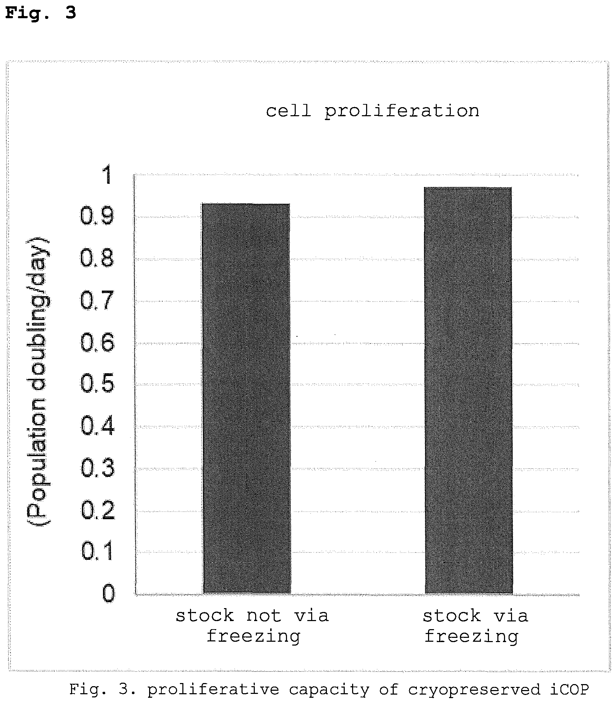

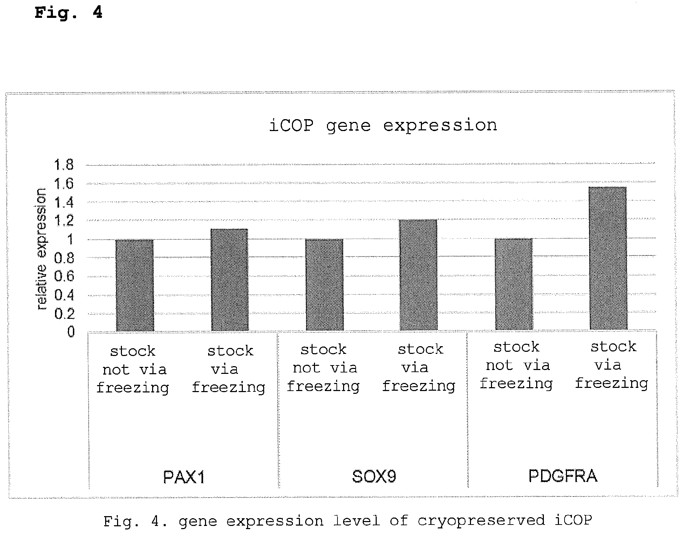

One day before the start of differentiation, 4×10⁶ cells of iPS cells were seeded on a 10 cm dish (BD FALCON) pre-coated with 0.5 µg/cm² iMatrix-511 (Nippi), and cultured in StemFit (registered trade mark) AK03N containing 10 µM Y-27632 in an incubator at 37°C,5% CO₂. The next day, the medium was replaced with differentiation 1 medium (8 mL) containing RPMI1640, 20% StemFit (registered trade mark) For Differentiation (Ajinomoto Co., Inc.), 30 ng/mL Activin A, 10 µM CHIR99021, 100 ng/mL bFGF, 0.3 µM LDN193189. The next day, the medium was replaced with differentiation 2 medium (8 mL) containing RPMI1640, 20% StemFit (registered trade mark) For Differentiation, 10 µM SB431542, 5 µM CHIR99021, 100 ng/mL bFGF, 0.3 µM LDN193189. The next day, the medium was replaced with differentiation 3 medium (8 mL) containing RPMI1640, 20% StemFit (registered trade mark) For Differentiation, 10 µM SB431542, 3 µM IWR-1, 0.3 µM PD0325901, 0.3 µM LDN193189. The next day, the medium was replaced with differentiation 4 medium (8 mL) containing RPMI1640, 20% StemFit (registered trade mark) For Differentiation, 3 µM IWR-1, 0.3 µM SAG. On days 4 and 5, the medium was exchanged with the differentiation 4 medium, and the cells on the 6th day were used as iCOP. The number of viable cells of iCOP was counted, and 2×10⁵ cells of iCOP were seeded on a 10 cm dish pre-coated with 0.5 µg/cm² iMatrix-511, and incubated in the iCOP proliferation medium containing DMEM/F12, 20% StemFit (registered trade mark) For Differentiation, 250 µM L-Ascorbic Acid 2-phosphate, 10 µM SB431542, 100 ng/mL bFGF, 0.3 µM SAG under the conditions of 37°C, 5% CO₂. The medium was exchanged once every two days. When the cells reached 90 - 100% confluence, the number of iCOP cells was counted, and the cells were divided into a group for continuing culture and a group for producing a frozen stock. To produce frozen stocks, 1×10⁶ cells were suspended in 1 mL STEM-CELLBANKER (registered trade mark) GMP grade (Zenoaq) and dispensed to a serum tube (IWAKI). Thereafter, the serum tube was put into BICELL (Japan Freezer) cooled to 4°C in advance, frozen in a -80°C deep freezer, and transferred to a -150°C deep freezer the next day. One week later, the frozen serum tube was thawed in a 37°C hot water bath, and 3×10⁵ cells of iCOP were seeded in a 10 cm dish pre-coated with 0.5 µg/cm² iMatrix-511, and the cells were cultured in the iCOP proliferation medium. The medium was exchanged once every two days and when the cells reached 90 - 100% confluence, the cells of iCOP were passaged, and cultured under the same conditions for 5 more days to obtain P3 iCOP. As a result, even though iCOP was cryopreserved, the proliferation capacity of iCOP did not decrease (Fig. 3), and the iCOP marker in P3 iCOP did not decrease (Fig. 4). From the above results, it was shown that iCOP can be cryopreserved.

### Example 4: Verification of bone differentiation potential of iCOP

In the following Experimental Example, iCOP was induced to differentiate into osteocyte, and the gene expression level of osteocyte markers was evaluated. As the iPS cells, the 1231A3 strain, 1210B2 strain, and 201B7 strain purchased from iPS Academia Japan were used.

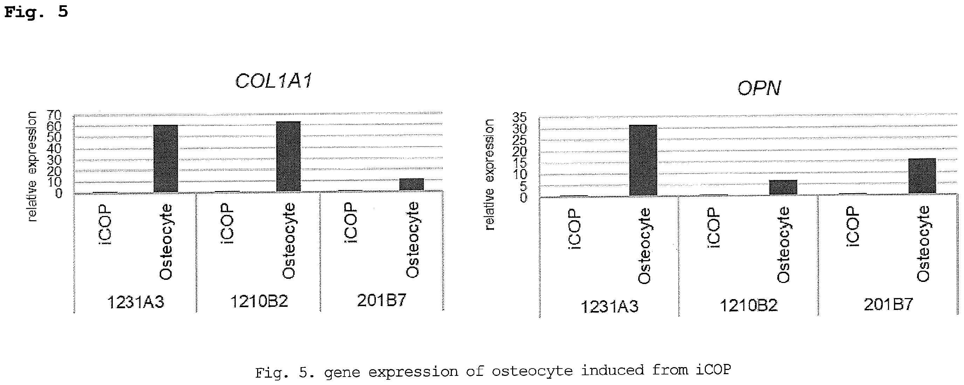

The iCOP cryopreserved in the 1st passage was cultivated and expansion cultured up to the 3rd passage in an iCOP proliferation medium containing DMEM/F12, 20% StemFit (registered trade mark) For Differentiation, 250 µM L-Ascorbic Acid 2-phosphate, 10 µM SB431542, 100 ng/mL bFGF, 0.3 µM SAG, 10 ng/mL PDGF-BB (Peprotech). When iCOP reached 90-100% confluence, the number of viable cells was counted and the cells were suspended in osteocyte differentiation medium containing 4.5 g/L Glucose containing DMEM (Nacalai Tesque), 20% StemFit (registered trade mark) For Differentiation, 250 µM L-Ascorbic Acid 2-phosphate, 100 nM dexamethasone (Sigma), 10 mM β-glycero phosphate (Nacalai Tesque). 2×10⁵ cells of iCOP were seeded on a 24-well plate (BD FALCON) pre-coated with 0.5 µg/cm² iMatrix-511 and allowed to stand in an incubator at 37°C, 5% CO₂. The medium was exchanged every other day. After culture for 18 days, the cells were collected and the gene expression level of the Osteocyte was evaluated by the real-time PCR method. As a result, the osteocyte marker (COL1A1 and OPN) genes were expressed in all cell lines, and it was shown that iCOP maintains the differentiation potential into osteocyte (Fig. 5).

### Example 5: Verification of syndetome differentiation potential of iCOP

In the following Experimental Example, iCOP was induced to differentiate into syndetome, and the gene expression level of syndetome markers was evaluated. As the iPS cells, the 1231A3 strain, 1210B2 strain, and 201B7 strain purchased from iPS Academia Japan were used.

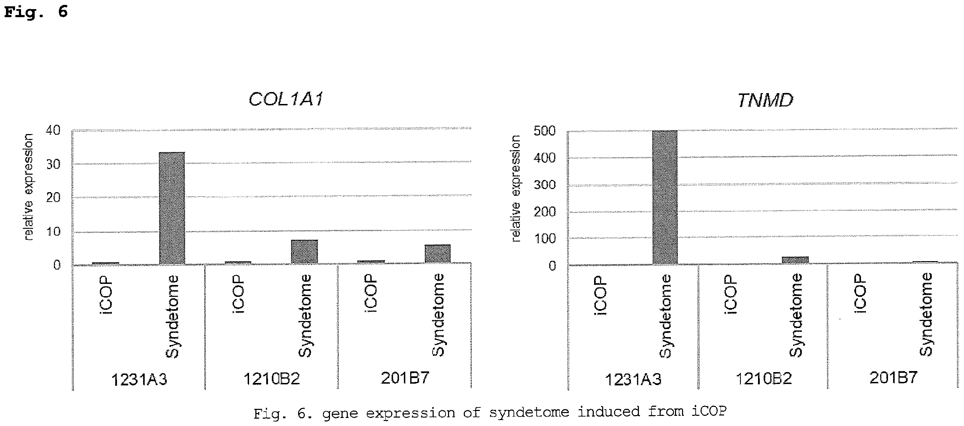

The iCOP cryopreserved in the 1st passage was cultivated and expansion cultured up to the 5th passage in an iCOP proliferation medium containing DMEM/F12, 20% StemFit (registered trade mark) For Differentiation, 250 µM L-Ascorbic Acid 2-phosphate, 10 µM SB431542, 100 ng/mL bFGF, and 0.3 µM SAG. When iCOP reached 90-100% confluence, the number of viable cells was counted and the cells were suspended in syndetome differentiation medium 1 containing DMEM/F12, 20% StemFit (registered trade mark) For Differentiation, 250 µM L-Ascorbic Acid 2-phosphate, 10 ng/mL TGF-β3 (Peprotech), 20 ng/mL FGF8b (Peprotech). 2.5×10⁵ cells of iCOP were seeded on a 24-well plate pre-coated with 0.5 µg/cm² iMatrix-511 and allowed to stand in an incubator at 37°C, 5% CO₂. Two days later, the medium was replaced with syndetome differentiation medium 2 containing DMEM/F12, 20% StemFit (registered trade mark) For Differentiation, 250 µM L-Ascorbic Acid 2-phosphate, 10 ng/mL TGF-β3, 10 ng/mL BMP-4 (R&D) and the cells were cultured for 6 more days. The medium exchange was performed every other day. After completion of the culture, the cells were collected and the gene expression level of the syndetome was evaluated by the real-time PCR method. As a result, the syndetome marker (COL1A1 and TNMD) genes were expressed in all cell lines, and it was shown that iCOP maintains the differentiation potential into syndetome (Fig. 6).

### Example 6: Expansion culture of iCOP by suspension culture

In the following Experimental Example, iCOP was proliferated by suspension culture, and its proliferative potential and differentiation potential into chondrocytes were evaluated. As the iPS cell, the 1231A3 strain purchased from iPS Academia Japan was used.

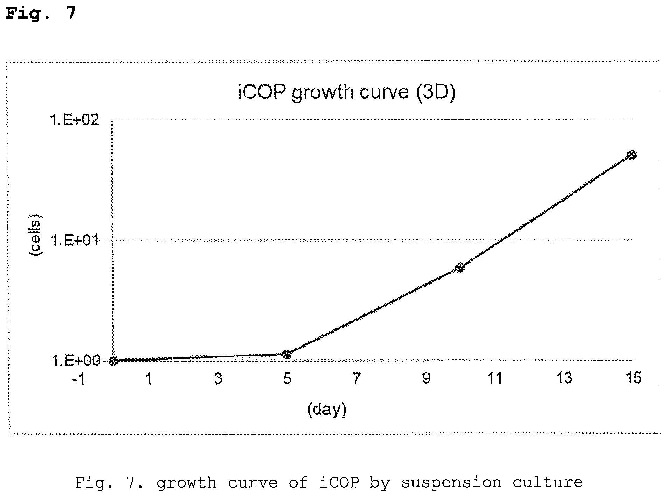

The number of viable cells of iCOP produced from iPS cells was measured, and the cells were suspended in iCOP proliferation medium containing DMEM/F12, 20% StemFit (registered trade mark) For Differentiation, 250 µM L-Ascorbic Acid 2-phosphate, 10 µM SB431542, 100 ng/mL bFGF, 0.3 µM SAG, and 10 ng/mL PDGF-BB. The cells were seeded at a cell density of 2×10⁵ cells/mL in a 5 mL single-use bioreactor (Biott), and the cells were cultured with stirring in an incubator at 37°C, 5% CO₂ at a stirring rate of 80 rpm. 70% of the medium was exchanged every other day. To evaluate the proliferative capacity of iCOP, iCOP was harvested from a single-use bioreactor, centrifuged, suspended in 1 mL of Accumax cell detachment solution (Merck), and allowed to stand for 10 min. The cells were suspended again until the cell aggregates were broken, centrifuged, suspended in iCOP proliferation medium, and the number of cells was measured. A similar operation was repeated every 5 days, and the growth curve until the 3rd passage was drawn. As a result, PD per day was 0.38 (Fig. 7). In addition, the gene expression of iCOP markers (PAX1, SOX9 and PDGFRA) was measured to find that the expression was maintained even after 3 passages (Table 3). Furthermore, iCOP in the 3rd passage was differentiated into chondrocytes. As a result, all chondrocyte genes were expressed (Table 4). From the above, it was shown that iCOP can be expansion cultured in a suspending state.

**[Table 3]**

| | iCOP | | | |
|---|---|---|---|---|
| | P0 | P1 | P2 | P3 |
| *PAX1* | + | + | + | + |
| *SOX9* | + | + | + | + |
| *PDGFRA* | + | + | + | + |

**[Table 4]**

| | chondrocyte |
|---|---|
| *COL2A1* | + |
| *ACAN* | + |
| *EPIPHYCAN* | + |

### Example 7: Identification of specific marker of iCOP

In the following Experimental Example, iCOP produced from iPS cell was analyzed using a next generation sequencer (NGS), and iCOP-specific markers were identified. As the iPS cell, the 1231A3 strain purchased from iPS Academia Japan was used.

One day before the start of differentiation, 1.6×10⁵ cells of iPS cells were seeded on 4 wells of a 24 well plate (BD FALCON) pre-coated with 0.5 µg/cm² iMatrix-511 (Nippi), and cultured in StemFit (registered trade mark) AK03N containing 10 µM Y-27632 in an incubator at 37°C, 5% CO₂. The next day, the medium was removed, and differentiation 1 medium containing RPMI1640, 20% StemFit (registered trade mark) For Differentiation (Ajinomoto Co., Inc.), 30 ng/mL Activin A, 10 µM CHIR99021, 100 ng/mL bFGF, 0.3 µM LDN193189 was added by 500 µL per 1 well. The next day, the medium was replaced with differentiation 2 medium (500 µL) containing RPMI1640, 20% StemFit (registered trade mark) For Differentiation, 10 µM SB431542, 5 µM CHIR99021, 100 ng/mL bFGF, 0.3 µM LDN193189. The next day, the medium was replaced with differentiation 3 medium (500 µL) containing RPMI1640, 20% StemFit (registered trade mark) For Differentiation, 10 µM SB431542, 3 µM IWR-1, 0.3 µM PD0325901, 0.3 µM LDN193189. The next day, the medium was replaced with differentiation 4 medium (500 µL) containing RPMI1640, 20% StemFit (registered trade mark) For Differentiation, 3 µM IWR-1, 0.3 µM SAG. On days 4 and 5, the medium was exchanged with the differentiation 4 medium, and the cells on day 6 were used as iCOP and the number of viable cells of iCOP was measured. The iCOP at this point was used as iCOP at the 0th passage, a cell suspension containing 1×10⁶ cells of the acquired cells was collected in a 1.5 mL Eppendorf tube. After centrifugation, the supernatant was removed, and the resulting pellets were cryopreserved as a sample for NGS analysis. In addition, 3×10⁵ cells of iCOP were seeded on a 10 cm dish pre-coated with 0.5 µg/cm² iMatrix-511, and incubated in the iCOP proliferation medium containing DMEM/F12, 20% StemFit (registered trade mark) For Differentiation, 250 µM L-Ascorbic Acid 2-phosphate, 10 µM SB431542, 100 ng/mL bFGF, 0.3 µM SAG under the conditions of 37°C, 5% CO₂. The medium was exchanged once every two days, and when the cells reached 90 - 100% confluence, the number of iCOP cells was counted. The iCOP at this point was used as iCOP at the 1st passage, a part of the acquired cells was passaged, and 1×10⁶ cells in the state of pellets were cryopreserved as a sample for NGS analysis. Passaging and acquisition of cell samples for NGS were repeated, whereby iCOP frozen samples at the 0th passage, 1st passage, 3rd passage, 5th passage, 8th passage, 10th passage were obtained. The obtained frozen samples were thawed and RNA was extracted using PureLink^{™} RNA Mini kit (Thermo Fischer Scientific). For quality check of the extracted RNA, RIN (RNA Integrity Number) was measured using a Bioanalyzer (Agilent RNA6000 Nano kit, Agilent). For library preparation of the samples for NGS analysis, KAPA Stranded mRNA-Seq Kit (KAPA Biosystems) and KAPA Unique Dual-Indexed Adapter Kit (KAPA Biosystems) were used. Bioanalyzer (Agilent DNA1000 kit, Agilent) was used for the quality check of the prepared library, and Qubit (registered trade mark) 2.0 Fluorometer (Thermo Fisher Scientific) and Qubit (registered trade mark) dsDNA HS assay kit (Thermo Fisher Scientific) were used for the concentration measurement. Using the prepared library, sequencing by NextSeq 500 System (illumina) was performed. FASTQ file was obtained from the BaseSpace (registered trade mark) Onsite (illumina) system, and trimming of the 3'-terminal with the Trimmomatic tool was performed on Bio-Linux. On Jupyter notebook, the quality improvement was confirmed by trimmomatic by FastQC, and the RNA-seq pipeline consisting of mapping to the human genome, read number counts, and normalization was performed, and using the output read count file, the expression variation gene (DEG) was analyzed using the TCC package. As a result of the analysis, 110 genes extracted as iCOP-specific marker genes are shown below.

*A2M, ABCA9, ACAT2, ADAMTS9, ADGRF5, AGTR2, AMER1, AMOT, ANGPT1, ARHGAP5-AS1 ARHGEF26, ATP8A1, BCHE, BEGAIN, BOC, C3ORF52, CACNA1G, CELF2, CLSPN, COL25A1 COL26A1, CORO1A, CRISPLD1, CTTNBP2, CYB5B, DLGAP1, DNAJC12, DUSP9, EBF1, EBF2 EFEMP1, ESCO2, EYA1, FAM35A, FAM78A, FAR2, FGFBP2, FGFR4, FKBP4, FRZB, GCNT4 GNG11, HAAO, HMGCS1, HS3ST5, ID4, IGDCC3, IRF8, KCNA6, KCNB2, KITLG, LGE5, LHCGR, LHFP, LPPR5, LUM, MAPK8IP2, MECOM, MEF2C, MEOX2, METTL7A, NAB1, NCALD, NGF, NGFR, NKAIN2, NPR1, NR2F1, OLFML1, PCDH18, PCDH19, PCSK9, PDE4D, PDGFRA, PEG10, PLVAP, PRELP, PRRT4, RARRES2, RBMS3, RBPMS2, RSPO3, RUNX1T1, SAMD5, SCARA5, SKIDA1, SLG12A2, SLC27A3, SLC39A8, SLC7A2, SLC8A3, SLCO1C1, SOX8, SPRED2, STARD8, STOM, SYNPO, TBX18, TMC6, TNRC6C-AS1, TRIM2, TRIM9, TRIP13, TSPAN15, UBE2N, UHRF1, UNC5C, VIT, ZBTB46, ZNF488*

Among the 110 genes, a gene group of cell surface antigens is shown below.

*ABCA9, ATP8A1, BOC, KITLG, NKAIN2, ADGRF5, AGTR2, CACNA1G, C3ORF52, COL25A1 CYB5B, FAR2, FGFR4, GCNT4, HS3ST5, IGDCC3, LGR5, LHFP, LHCGR, NPR1, NGFR, PLVAP PDGFRA, KCNA6, KCNB2, PCDH18, PCDH19, SCARA5, SLC12A2, SLC27A3, SLC39A8, SLC7A2 SLC8A3, SLCO1C1, STOM, TSPAN15, TMC6, UNC5C*

### [Industrial Applicability]

According to the present invention, expansion culture of a cartilage or bone progenitor cell becomes possible. The period for inducing differentiation into chondrocytes or osteocytes can be drastically shortened by expansion culturing and stocking the progenitor cell in advance. In addition, an extremely large amount of chondrocytes or osteocytes can be produced. Therefore, chondrocytes in an amount sufficient for use in the treatment of cartilage diseases or bone diseases can be rapidly produced by the present invention.

This application is based on a patent application No. 2019-094878 filed in Japan (filing date: May 20, 2019), the contents of which are incorporated in full herein.

## Claims

1. A method for expansion culture of a cartilage or bone progenitor cell, comprising a step of culturing a cartilage or bone progenitor cell in a medium comprising a TGF-β signal inhibitor and FGF.

2. The method according to claim 1, wherein the cartilage or bone progenitor cell is a cell that expresses PDGFRA, SOX9, or PAX1.

3. The method according to claim 1 or 2, wherein the TGF-β signal inhibitor is SB431542.

4. The method according to any one of claims 1 to 3, wherein the FGF is bFGF.

5. The method according to any one of claims 1 to 4, wherein the cartilage or bone progenitor cell is derived from a pluripotent stem cell.

6. The method according to any one of claims 1 to 5, wherein the cartilage or bone progenitor cell is obtained by suspension culture of a pluripotent stem cell.

7. The method according to any one of claims 1 to 6, wherein the expansion culture is suspension culture.

8. A kit for expansion culture of a cartilage or bone progenitor cell, comprising a basal medium, a TGF-β signal inhibitor, and FGF.

9. The kit according to claim 8, wherein the TGF-β signal inhibitor is SB431542.

10. The kit according to claim 8 or 9, wherein the FGF is bFGF.

11. A method for producing a chondrocyte or osteocyte, comprising a step of inducing differentiation of a cartilage or bone progenitor cell obtained by the method according to any one of claims 1 to 7 into a chondrocyte or osteocyte.

12. A method for producing a syndetome, comprising a step of inducing differentiation of a cartilage or bone progenitor cell obtained by the method according to any one of claims 1 to 7 into a syndetome.
